# EUROPEAN PATENT APPLICATION

(11) **EP 1 118 340 A1**
(43) Date of publication of application: **25.07.2001**
(21) Application number: 00100258.3
(22) Date of filing: 18.01.2000
(51) Int. Cl.: A61L 15/36, A61F 13/15, B32B 9/00

(54) **Articles having an odour control system comprising lactic-acid producing micro-organisms and an odour absorbing agent**

(71) Applicant: THE PROCTER & GAMBLE COMPANY, Cincinnati, Ohio 45202 (US)
(72) Inventor: Di Cintio, Achille, 65126 Pescara (IT); Carlucci, Giovanni, 66100 Chieti (IT); Pesce, Antonella, 65120 Pescara (IT)
(74) Representative: Kremer, Véronique Marie Joséphine

(57) **Abstract**

The present invention relates to articles suitable for controlling odours, especially odours associated with bodily fluids, which comprise lactic acid producing microorganisms, preferably spore-forming lactic acid producing micro-organisms, together with an odour absorbing agent, preferably silica and/or zeolite. In a preferred embodiement the articles further comprise an absorbing gelling material.

## Description

### Field of the Invention

This invention relates to articles, such as absorbent articles, for controlling odours, especially odours associated with bodily fluids, comprising lactic acid producing micro-organisms together with an odour absorbing agent.

### Background of the Invention

Malodours may be present in the environment from numerous sources both animate and inanimate. Many products and articles are available which aim to avoid or minimise the detection of such odours. In particular, it is particularly desirable to provide odour control materials to address the malodours which are generated by the human body, or from bodily fluids such as perspiration, urine, faeces, menstrual fluids, vaginal fluids and the like.

Articles like absorbent articles for example are designed to be worn by humans to absorb bodily fluids, such as urine, menstrual fluid and perspiration, etc. Examples of absorbent articles include sanitary napkins, pantiliners, disposable diapers, incontinence pads, tampons, perspiration pads, nursing pads and the like.

In use, the absorbent articles are known to acquire a variety of compounds, for example volatile fatty acids (e.g. isovaleric acid), ammonia, amines (e.g. triethylamine), sulphur containing compounds (e.g. mercaptans, sulphides), alcohols, ketones and aldehydes (e.g., furaldehyde) which release unpleasant odours. These compounds may be present in the bodily fluid or may be developed by chemical reactions and/or any fluid degradation mechanisms once the bodily fluid is absorbed into the absorbent article like for example a feminine pad. In addition bodily fluids usually contain microorganisms and/or enzymes that can also generate malodorous by products as a result of degradation mechanisms like putrefactive degradation, acid degradation, proteins degradation, fat degradation and the like. Unpleasant odours which emanate from absorbent pads when in use may make the wearer feel self conscious.

Various odour control materials have been disclosed in the art to combat some of the unpleasant odours referred to above. Indeed solutions have been provided that use different technical approaches like masking, i.e., covering the odour with a perfume, or absorbing the odour already present in the bodily fluids and those generated after degradation.

Most of the focus in the prior art is found on the odour absorption technology. Examples of these types of compounds include activated carbons, clays, zeolites, silicates, starches, cyclodextrine, ion exchange resins and various mixture thereof as for example described in EP-A-348 978, EP-A-510 619, WO 91/12029, WO 91/11977, WO89/02698, and/or WO 91/12030. All of these types of odour absorbing agents are believed to control odour by mechanisms whereby the malodorous compounds and their precursors are physically absorbed by the agents and thus such agents hinder the exit of the odour from articles like absorbent articles. However, such mechanisms are not completely effective as the formation of the odour itself is not prevented and thus odour detection is not completely avoided.

Thus although these materials provide some control of odours associated with bodily fluids, there still exists a need of further improvement in terms of odour control over a wide range of malodorous compounds.

It is an object of the present invention to provide effective odour control over a wide range of malodours. More particularly, it is an object of the present invention to provide articles, especially disposable absorbent articles, which deliver outstanding odour control over a broad spectrum of malodours.

It has now been found that the above needs can be addressed by combining an odour absorbing agent together with lactic acid producing micro-organisms, as the odour control system for an article, preferably a disposable absorbent article.

It has been surprisingly discovered that the combination of an odour absorbing agent, preferably silicate and/or zeolite, together with lactic acid producing micro-organisms in an article, like an absorbent article typically coming into contact with bodily fluids, results in a synergistic effect in terms of odour control. Indeed this combination gives more odour reduction than the odour reduction associated with the use of one of these two classes of ingredients alone at the same total level (either the odour absorbing agent alone or the lactic acid producing micro-organisms alone) in an absorbent article when in contact with bodily fluids.

Actually the combination of the lactic acid producing micro-organisms with an odour absorbing agent in an article herein allows to combine odour control mechanisms by which the overall malodour detection is synergistically reduced or even prevented.

The combination herein allows odour control over a wide range of malodours which would otherwise not have been fully controlled by one of these two classes of ingredients used alone. More surprisingly, the presence of the lactic acid producing micro-organisms allows to increase the effectiveness of the odour absorbing agent. Indeed the lactic acid producing micro-organisms control the growth of pathogenic micro-organisms and as a consequence the amount of malodorous compounds produced by these micro-organisms. In other words, the lactic acid producing micro-organisms prevent the formation of malodour, thereby reducing the total amount of malodour to be controlled. This allows the odour absorbing agent to work in reduced amount of active. Actually this results in a more effective as well as a sustained use of the odour absorbing agent herein. Indeed the saturation point of the odour absorbing agent when used in association with the micro-organisms herein will be reached after prolonged periods of use, typically after prolonged wearing time of an absorbent article (pantiliner, pad) coming into contact with bodily fluid, as compared to when used alone in the same conditions. Advantageously it is believed that the odour absorbing agents also help the lactic-acid producing micro-organisms in reducing malodor by adsorbing the odor of head space (space between the absorbent article and the urogenital surface) and the malodorous components of fluids that are not controlled by these microorganisms.

The lactic acid producing microorganisms used herein have the ability to deliver antagonistic properties against undesirable pathogens which are known to cause unpleasant odors like for example the bacteria belonging to the family Enterobacteriaceae, e.g., Proteus mirabilis, Proteus vulgaris, Echerichia coli and Klebsiella. The metabolic activity of such pathogen bacteria which aims for satisfying the bacteria needs for energy and proliferation, leads to degraded odorous compounds as by products which are among others low molecular weight fatty acids, amines, mercaptan, indoles, ammonia, sulfide and the like. The antagonistic microorganisms according to the present invention inhibit the growth of such pathogen microorganisms, by competing for substrate and generating a non conducive acidic environment by changing the pH to more acidic values (e.g., pH 4 to 4.5). The antagonistic properties of the lactic-acid producing microorganisms according to the present invention are also partly denoted their ability of producing other antimetabolites, like enzymes (e.g., lactoperoxidases), toxins, carbon dioxide, peroxides or antibiotics, so-called bacteriocines.

Additionally beside the odor formation prevention benefits other benefits are associated to the antagonistic microorganisms used herein. Indeed the competitive inhibition of pathogen microorganisms will also translate in reduction of the risk of infection in the urogenital environment of a user and/or in reduction of the risk of skin infection.

In a preferred embodiment the lactic-acid producing micro-organisms used herein are spore-forming lactic producing microorganisms, preferably *Lactobacillus sporogenes.* These micro-organisms have the ability to create very quickly an environment that is not suitable for the growth of pathogens. This is due to the rapid growth, high yield and reproducibility of such microorganisms in comparison to other lactic acid producing bacteria like *Lactobacillus acidophilus.* Furthermore these micro-organisms are spore forming micro-organisms, i.e., they are able to survive longer and reproduce themselves in comparison to non spore-forming micro-organisms. In other words, by the formation of spores these micro-organisms can germinate and re-germinate in time sequence in line with the bodily discharge (growth media) into an absorbent article, thereby ensuring long-lasting antagonistic properties against pathogens, typically long lasting odour control.

In the most preferred embodiment of the present invention the dormant form of the spore-forming lactic acid producing microorganisms, i.e., spores (preferably *L. sporogenes* spores) are used as the lactic acid producing micro-organisms herein. These spores have the ability to germinate in the living form of the micro-organisms which exhibit antagonistic properties against undesirable pathogens. The use of such spores in the articles herein provides improved stability both during storage and use. Indeed the use of the spores herein provides an effective odour controlling article which can be stored for long periods of time before its actual use, without risking that the odour control ability of the article in use is impaired. Actually, the use of these spores able storage stability upon prolonged periods of time up to the time the article is used, i.e., is applied on the external surface of a human or animal body where it typically comes into contact with bodily fluids, and undergoes environmental changes that will create favourable environmental condition for the germination of the spores. Indeed the genetic identity, the lactic acid producing ability and the viability of the antagonistic microorganisms when using such spores are not impaired upon prolonged periods of storage and are maintained upon prolonged periods of use.

Advantageously, in the embodiment herein wherein the spores as described herein are used the articles according to the present invention retain their antagonistic properties against pathogens like the odour control capacity up to the time their are used. Thus the present invention provides articles with effective odour controlling ability, while using reduced total amount of odour control active like the spores herein.

A further advantage associated with the disposable absorbent articles of the present invention like pantiliners or pads, is a better feeling and more acceptable cleanness level in use. Indeed, it has been found that the microorganisms herein allow gelification of the bodily fluid discharge, thereby facilitating the fluid transport into the article. In a preferred embodiment the microorganisms are located in the core of the absorbent article, thereby changing the physical properties of bodily fluids. Such gelification will have the benefits of reducing the rewetting of the topsheet and the soiling through as might otherwise result from squeezing the article as a consequence of legs movement of a person wearing the article. This translates in consumer noticeable dryness and cleanness benefit. Thus the present invention provides a disposable absorbent article which combines outstanding odor control over a broad spectrum of malodor and effective level of protection upon contact with bodily fluid.

In a preferred embodiment herein the disposable absorbent articles herein have an apertured polymeric film topsheet. This topsheet contributes to further improve the odor control benefit.

In another preferred embodiment herein the disposable absorbent articles herein have a breathable backsheet. This contributes to a further improved odor control benefit. Even more preferred herein the disposable absorbent articles have both a breathable backsheet and an apertured polymeric film topsheet.

Whereas the present invention is preferably directed to disposable articles like pantiliners, feminine napkins, incontinent pads, diapers, tampons, interlabial pads, perspiration pads, surgical pads, breast pads, human or animal waste management devices and the like, other articles may include the odour control system as described herein too for the purpose of effective odour control. Indeed, other applications include other articles designed to be worn in contact with the body such as clothing, bandages, thermal pads, acne pads, cold pads, compresses, surgical pads/dressings and the like, body cleansing articles like impregnated wipes/tissues (e.g. baby wipes, wipes for feminine intimate hygiene), articles for absorbing perspiration such as shoe insoles, shirt inserts, and the like, and articles for animals like litters and the like.

### Background art of the invention

Articles are available which comprise probiotic agents like lactic-acid producing micro-organisms. For example International Patent Application WO 92/13577 describes a tampon or sanitary napkin that has been impregnated with a culture of lactic-acid producing bacteria, preferably of the genus Pediococcus, isolated from healthy individuals. WO 97/02846 discloses an absorbent article with antagonistic microorganisms selected from the family Lactobacillaceae, preferably from the genera Lactobacillus or Lactococcus. WO99/17813 discloses an absorbent article with a suspension of lactic acid bacteria from the genera Lactobacillus, Lactococcus or Pediococcus, the article being dried after application of the suspension, to a moisture content of less than 10% calculated as percentage of weight of the absorbent core in the article.

None of these references does disclose the presence of any odour absorbing agent according to the present invention let alone the preferred ones like zeolite and/or silicate.

### Summary of the Invention

The present invention relates to an article, preferably a disposable absorbent article, for controlling odours, preferably odours associated with bodily fluids, comprising lactic acid producing micro-organisms and at least one odour absorbing agent. In a preferred embodiment of the invention the article also comprises an absorbing gelling material.

### Detailed description of the Invention

The articles according to the present invention, for controlling odour, especially odour associated with bodily fluids, comprise as an essential element lactic acid producing micro-organisms and an odour absorbing agent.

By "article" it is meant herein any tridimentional solid material being able to receive/carry an odour control system as described herein comprising lactic acid producing micro-organisms and at least one odour absorbing agent. The term 'disposable' is used herein to describe articles which are not intended to be launched or otherwise restored or reused as an article (i.e., they are intended to be discarded after a single use and, preferably to be recycled, composted or otherwise disposed of in an environmentally compatible manner).

Preferred articles according to the present invention are disposable absorbent articles that are designed to be worn in contact with the body of a user and to receive fluids discharged from the body, such as pantiliners, sanitary napkins, catamenials, incontinence inserts/pads, diapers, tampons, interlabial pads/inserts, breast pads, human or animal waste management devices and the like. Typically such human urine or faecal management devices comprise a bag having an aperture and a flange surrounding the aperture for preferably adhesive attachment to the urogential area and/or the perianal area of a wearer. Any faecal or urine management device known in the art is suitable for use herein. Such devices are described in for example WO 99/00084 to WO 99/00092. Other articles suitable according to the present invention also include other articles designed to be worn in contact with the body such as clothing, bandages, thermal pads, acne pads, cold pads, compresses, surgical pads/dressings and the like, articles for absorbing perspiration such as shoe insoles, shirt inserts, perspiration pads and the like, body cleansing articles like impregnated wipes/tissues (e.g. baby wipes, wipes for feminine intimate hygiene), and the like, and articles for animals like litters and the like.

By "bodily fluids" it is meant herein any fluid produced by human or animal body occurring naturally or accidentally like for instance in the case of skin cutting, including for instance perspiration, urine, menstrual fluids, faeces, vaginal secretions and the like.

### The lactic acid producing micro-organisms

According to the present invention the articles comprise as an essential component a lactic acid producing microorganism or a mixture thereof.

Suitable lactic acid producing microorganisms for use herein also called antagonistic microorganisms are microorganisms that exhibit antagonistic properties against undesirable strain of microorganisms by releasing amongst other metabolites, lactic acid. The microorganisms that exhibit such antagonistic properties may be bacteria or other microorganisms for instance fungi.

Suitable lactic acid producing bacteria (antagonistic bacteria) for use herein include those belonging to the genera Lactobacillus, Lactococcus, Pedioccocus and/or Leuconostoc, and preferably the species *Lactobacillus acidophilus, Lactobacillus curvatus, Lactobacillus plantarum*, *Lactobacillus jenseni, Lactobacillus casei, Lactobacillus fermentum*, *Lactococcus lactis, Pedioccocus acidilacti*, *Pedioccocus pentosaceus*, *Pedioccocus urinae* and/or *Leuconostoc mesenteroides*.

Highly preferred lactic acid-producing microorganisms for use herein are the spore-forming lactic acid-producing microorganisms. These preferred microorganisms have the ability to survive in hostile environment in spore form (dormant form). Sporulation is the development in microorganisms of bodies each wrapped in a protective coat (a natural process of microencapsulation in a calcium-dipicolinic acid-peptidoglycan complex). Under favorable conditions, the spores germinate into viable bacilli (living form) and carry on their life activities. Suitable spore-forming lactic acid-producing microorganisms for use herein include the microorganisms belonging to the family Sporolactobacillaceae, particularly the genus Sporolactobacillus (e.g., *S*. *inulinus*). Highly preferred for use herein is the species *Lactobacillus sporogenes* (in its living and/or dormant form).

*L. sporogenes* was first isolated from green malt and described in 1933 by L.M. Horowitz-Wlassowa and N.W. Nowotelnow. It was submitted as L. *sporogenes* in the fifth edition (1939) of 'Bergey's manual of Determinative Bacteriology' as well as mentioned in recognized scientific publication, Korean J. Appl. Microb. & Bioengin. (1985) 13:185-190, J. Pharmaceut. Soc. Korea (1977) vol XXIII, 1-Feb, 473-474. *L*. *sporogenes* was transferred to *Bacillus coagulans* in the seventh edition of 'Bergey's manual of Determinative Bacteriology' due to simplification in cataloguing. However in honour of the original discoverers the name *L*. *sporogenes* is used widely. Reference is also made to the taxonomical classification of *Sporolactobacillus* in L'integratore Nutrizionale 2 (1) 1999, Stabilita' di integratori con Sporolactobacillus, classificazione tassonomica by L. Marossi and all.

According to the Eighth Edition of Bergey's Manual of Determinative Bacteriology, "various spore-bearing rods which produce lactic acid, are facultative or aerobic and catalase positive, have generally and correctly been assigned to the genus *Bacillus'.* The characteristics of *L*. *sporogenes* as cited in 'Bergey's Manual of Determinative Bacteriology' (seventh edition) and other sources are "gram positive spore-forming rods 0.9 by 3.0 to 5.0 micron size, aerobic to microaerophilic, producing L (+) lactic acid homofermentatively". L. *Sporogenes* also release other metabolites like carbon dioxide, diacetyl, bacteriocins, lacto-peroxidase.

Since *L. Sporogenes* exhibits characteristics typical of both genera *Lactobacillus* and *Bacillus,* its taxonomic position between the families *Lactobacillaceae* and *Bacillaceae* has often been discussed. This along with the fact that there is no universally accepted official classification leaves room for controversy in the nomenclature. More information about *L.sporogenes* is available from the commercial brochure 69/107, incorporated herein by reference, of Sochim International s.p.a. Milano, a supplier of the spore form of *L. Sporogenes*.

Some authors refer to *L.sporogenes* as *Bacillus coagulans*. *Bacillus coagulans* Hammer deposited as *Lactobacillus sporogenes* by Kabushiki Kaisha Naruse Fermentation Research Laboratory is commercially available under ATCC number 31284 (Internet information source : http://www.atcc.org/). ATCC stands for American Type Culture Collection.

*Lactobacillus sporogenes*, are preferred herein as they have the ability to create very quickly (faster than for example non-spore forming Lactobacillus) an environment that is not suitable for the growth of pathogens. This is due to the rapid growth, high yield and reproducibility of these micro-organisms in comparison to other lactic acid producing bacteria like *Lactobacillus acidophilus.* For example *L. sporogenes* needs 30 minutes for one generation while *L. acidophilus* needs 80 minutes. Furthermore these micro-organisms are spore forming lactic acid producing micro-organisms, i.e., they are able to survive longer and reproduce themselves in comparison to non spore-forming micro-organisms. Indeed in contrast to non-spore forming bacteria, *L*. *sporogenes* (living form) are transformed in spores (dormant form) when the substrate is reduced (in absence or reduced amount of bodily fluid discharge) and germinate again upon further bodily fluid discharge, i.e., reproduce themselves again through the spore form. In other words, by the formation of spores these micro-organisms can germinate and re-germinate in time sequence in line with the bodily fluid discharge (growth media) into the article, thereby ensuring long-lasting antagonistic properties against pathogens, typically long lasting odour control.

It is understood herein that the lactic acid producing micro-organisms according to the present invention also include the spores (dormant form) of the micro-organisms (living form) when referring to *Sporolactobacillus* and *L*. *sporogenes*. These spores are typically activated due to the presence of substrate, temperature increase and pH change. The optimum growth temperature range for the spores of *L sporogenes* is between 30°C and 50°C and the optimum pH range is from 5.0 to 6.5.

*L.sporogenes* spores are ellipsoidal bodies measuring 0.9 to 1.2 by 1.0 to 1.7 microns. These spores are commercially available in a white to greyish powder form. *L. sporogenes* spores powder is commercially available under the name LACTOSPORE® from SABINSA CORPORATION (Sochim international s.p.a, Milano). 1 gram of LACTOSPORE® corresponds to 15^{*}10⁹ spores and thus to 15*10⁹ cfu (colony forming unit) of *L*. *sporogenes*.

In the embodiment of the present invention where the spores as described herein before are used, the articles, preferably disposable absorbent articles, can be stored for longer periods of time before their actual use and still contain their whole antagonistic capacity up to the time the articles are used. *Indeed L. sporogenes* cells (in spore form) are protected from destruction by environmental factors by the naturally-present microencapsulation system, the spore coat. These spores are activated by environmental changes like pH and temperature changes and availability of substrate. For instance when the article is contacted with the skin or the urogenital zone of a wearer, the temperature increases (from room temperature to 30°C-35°C) and substrates like perspiration (pH 6), menstrual fluid discharge (pH 6.5), and/or urine discharge (pH 6.4) are provided. Such conditions will active the germination/re-germination of the spores. The spore coat imbibe water, swell and the increased water content will cause a rise in the metabolic rate of the sporulated bacilli. Outgrowths will begin to protrude from the spore-coats. The outgrown cells germinate and transform into viable vegetative cell (also called 'living form' herein). The living form begin to proliferate multiplying rapidly. These living forms continue their metabolic activities producing lactic acid and other metabolites which render the environmental non-conducive for the growth of harmful pathogenic micro-organisms. The germination process and more particularly the re-germination process (i.e., subsequent germination after the micro-organisms have been transformed into spores upon decrease of substrate availability) will be influenced by the body discharge in the article, leading to a somehow controlled germination/re-germination process on demand. In other words all the spores will not germinate with the same kinetic but the germination will be in relation to the body fluid discharge on the article. This will contribute to further sustain the effective odour control activity.

The lactic acid producing microorganisms (living form) are typically used in a freeze-dried form. Their isolation process follows known routine processes for the isolation of pure cultures. The isolated pure cultures are then typed according to known methods, e.g., API. The desired lactic acid producing bacteria are then cultivated in a fermentor in a manner known per se, are separated from the medium using a separator or a centrifuge, are freeze-dried in a manner known per se and ground to a fine powder. The bacterial concentrate in powder so obtained is then typically mixed with a fermentable carbohydrate, e.g., glucose, to a desired concentration. Such powder is then ready for use in the absorbent article. Alternatively in some case it is possible to add the living bacteria to the absorbent article and then carry out a freeze drying thereof. Another powder available form of the microorganisms herein is a lyophilized form.

Typically the number of lactic acid producing microorganisms per article, typical disposable absorbent article, exceeds 10² cfu, preferably exceeds 10⁴ cfu, more preferably is between 10⁵ cfu to 10¹² cfu and most preferably from 10⁶ to 10¹⁰ cfu.

Pathogens bacteria that cause bad smell may belong to, for example, the families Enterobacteriaceae, Ascomycetes, Pseudomonadaceae and Micrococcaceae and the genus Streptococcus. Examples of species and genera are Pseudomonas, Candida albicans, Escherichia coli, Proteus mirabilis, Proteus vulgaris, Enterococcus, Klebsiella, Staphylococcus and Streptococcus.

The particularities of the antagonistic microorganisms herein is that they inhibit other microorganisms by competing for substrates, forming metabolites like lactic acid, enzymes like lacto-peroxidases, toxins, carbon dioxide, peroxides or antibiotics, so-called bacteriocines. They have the ability to sustain the growth and reduce the patogenicity of many pathogens like ones mentioned herein before and especially Proteus, Pseudomonas, Echerichia, Klebsiella, Enterococcus, Staphylococcus, Streptococcus and Candida.

It is speculated that the production of lactic acid lowers the pH to 4-5, thereby inhibiting the growth of putrefactive organisms like E.coli, which require a higher optimum pH (typically 6 to 7) for favourable growth conditions. Furthermore undissociated lactic acid has the tendency to penetrate the membrane of pathogens, lowering their intracellular pH and/or interfering with their metabolic processes such as oxidative phosphorylation, thereby inhibiting the growth of such pathogens.

Other metabolites further contribute to inhibit the growth of pathogens. For example carbon dioxide is believed to reduce membrane permeability. Hydrogen peroxide / Lactoperoxidase are believed to oxidise basic proteins and to destroy the "enzymes factories" (ribosomes) of pathogens. Bacteriocins are proteins or protein complexes with bactericidal activity. Indeed, bacteriocins have the ability to link to particular receptors on the cell wall of microorganisms, thereby affecting the functionality of the cell wall/membranes. Bacteriocins are also able to affect DNA-synthesis and protein synthesis.

The particularity of the antagonistic microorganisms herein is that they are naturally occurring microorganisms that are non-toxic and do not have any negative biological effect on humans.

One advantage afforded by the use of antagonistic microorganisms is that there is avoided an undesired selection pressure on the micro environment, such as favoring potential desease-promoting microorganisms and therewith the risk of developing pathogenic strains that are resistant to antibiotics and chemopharmaceutical preparations. Since the antimicrobial system is based on a natural, biological process, there is less risk of environmental ecological and toxic disturbances.

It has now surprisingly been found that the lactic acid producing micro-organisms, preferably the spore-forming lactic acid producing microorganisms like *L. sporogenes* when used on top of the odour absorbing agent as described herein provide significant improved odour control capacity versus odour associated with bodily fluids. More particularly their combination results in a synergistic odour reduction, as compared to the odour reduction obtained for each of these two classes of odour controlling agents taken alone, when used at the same total level, in a same article (typically a disposable absorbent article) coming into contact with bodily fluids.

Without to be bound by theory, it is speculated that the antagonistic micro-organisms herein reduce or even prevent the generation of malodours by blocking the microbial and/or enzymatic activity of pathogenic micro-organisms responsible for the formation of malodorous compounds. This allows the odour absorbing agent to work in reduced amount of active. Actually this results in a more effective as well as a sustained use of the odour absorbing agent herein. Indeed the saturation point of the odour absorbing agent when used in association with the micro-organisms herein will be reached after prolonged periods of use, typically after prolonged wearing time of an absorbent article (pantiliner, pad) coming into contact with bodily fluid, as compared to when used alone (in absence of the micro-organisms according to the present invention) in the same conditions. Advantageously it is believed that the odour absorbing agents also help the lactic-acid producing micro-organisms in reducing malodor by adsorbing the odor of head space (space between the absorbent article and the urogenital surface) and the malodorous components of bodily fluids that do not get into contact or are not controlled by the lactic-acid producing micro-organisms.

Advantageously it has further been observed that the microorganisms herein have the advantage of changing the physical properties of bodily fluid. Indeed a gelification of the bodily fluid is obtained when the fluid comes into contact with the microorganisms herein. This results in reduced leakage/wet through of the absorbent articles as well as in improved dryness of the wearer facing surface. When talking about reduced leakage and/or improved dryness reference is made to a same absorbent article in absence of such microorganisms. As used herein the tospheet provides said wearer facing surface. Improved dryness and cleanness is particularly noticed on the wearer facing surface in those embodiments wherein the lactic acid producing microorganisms are located in the absorbent core.

Advantageously the present invention provides absorbent articles which upon contact with bodily fluid, provide effective and long lasting odor control, but also effective level of protection of the absorbent articles, resulting in consumer noticeable benefits like dryness and cleanness.

Without to be bound by theory, it is speculated that the cause of this gelification is the denaturation of the proteins. Indeed the lactic acid-producing microorganisms herein, through the glycogen fermentation, produce lactic acid. As proteins are sensitive to pH, the presence of lactic acid causes the soluble proteins contained into the bodily fluid to turn into insoluble form. This creates a sort of tri-dimensional net of molecules trapping globules, minerals, fats which results in the so called gelification of the bodily fluid.

### The odour absorbing agents

The articles according to the present invention further comprise on top of the lactic acid producing micro-organisms described herein before, at least one odour absorbing agent or a mixture thereof.

Suitable odour absorbing agents for use herein include activated carbons, clays, zeolites, silicas, kieselguhr, starches, cyclodextrin, ion exchange resins and the like. Preferred herein are silicas and/or zeolites. Highly preferred herein is a mixture of silica and zeolite.

Cyclodextrin and derivatives thereof may be used as described in US 5429628. Ion exchange resins may be used such as those described in US 4 289 513 and US 3340875.

Typically, the articles like disposable absorbent articles herein comprise from 20 to 600 gm⁻², more preferably from 40 to 500 gm⁻², most preferably from 100 to 400 gm⁻², of the odor absorbing agent or a mixture thereof.

### Silica

Particularly suitable herein as an odor absorbing agent is silica. Silica, i.e. silicon dioxide SiO₂ exists in a variety of crystalline forms and amorphous modifications, any of which are suitable for use herein. In particular, silicas having a high surface area or in agglomerated form are preferred. Silica molecular sieves are not considered to be within the definition of silica as used herein. Preferably the silica is in a highly purified form such that is contains at least 90%, preferably 95%, more preferably 99% silicon dioxide. Most preferably the silica is silica gel having a 100% silica content. Alternatively, the silica may be provided from other sources such as metal silicates including sodium silicate.

According to the present invention the absorbent articles typically may comprise from 0 to 300 gm⁻², more preferably from 40 to 250 gm⁻² most preferably from 60 to 200 gm⁻², of silica based on 100% purity or a mixture thereof.

### Zeolite

Another particularly suitable odour absorbing agent herein is zeolite. The use and manufacture of zeolite material is well know in the literature and is described in the following reference texts: ZEOLITE SYNTHESIS, ACS Symposium Series 398, Eds. M. L. Occelli and H. E Robson (1989) pages 2-7; ZEOLITE MOLECULAR SIEVES, Structure, Chemistry and Use, by D. W. Breck, John Wiley and Sons (1974) pages 245-250, 313-314 and 348-352; MODERN APPLICATIONS OF MOLECULAR SIEVE ZEOLITES, Ph.D. Dissertation of S. M. Kuznicki, U. of Utah (1980), available from University of Microfilms International, Ann Arbor, Michigan, pages 2-8.

Zeolites are crystalline aluminosilicates of group IA and group IIA elements such as Na, K, Mn, Ca and are chemically represented by the empirical formula :

M_{2/n}O . Al₂O₃. ySiO₂. wH₂O

where y is 2 or greater, n is the cation valence, and w is the water content in the voids of the zeolite.

Structurally, zeolites are complex, crystalline inorganic polymers based on an infinitely extending framework of AlO₄ and SiO₄ tetrahedra linked to each other by sharing of oxygen ions. This framework structure contains channels or interconnected voids that are occupied by the cations and water molecules.

The structural formula of a zeolite is based on the crystal unit cell, the smallest unit of structure, represented by

M_{x/n} [(AlO₂)ₓ (SiO₂)_{y}]. wH₂O

where n is the valence of cation M, w is the number of water molecules per unit cell, x and y are the total number of tedrahedra per unit cell, y/x usually having values of 1-5.

Zeolites may be naturally derived or synthetically manufactured. The synthetic zeolites being preferred for use herein. Suitable zeolites for use herein include zeolite A, zeolite P, zeolite Y, zeolite X, zeolite DAY, zeolite ZSM-5, or mixtures thereof. Most preferred is zeolite A.

According to the present invention the zeolite is preferably hydrophobic. This is typically achieved by increasing the molar ratio of the SiO₂ to AlO₂ content such that the ratio of x to y is at least 1, preferably from 1 to 500, most preferably from 1 to 6. According to the present invention the absorbent articles typically comprise from 0 to 300 gm⁻², more preferably from 40 to 250 gm⁻² most preferably from 60 to 200 gm⁻², of zeolite based on 100% purity or a mixture thereof.
In a preferred embodiment herein the absorbent article comprises silica together with zeolite as the odour absorbing agents in a weight ratio of silica to zeolite in a range of from 1:5 to 5:1, preferably from 3:1 to 1:3 and most preferably about 1:1.

### Carbon material

The carbon material suitable for employment herein is the material well known in the art as an absorber for organic molecules and/or air purification purposes. Carbon suitable for use herein is available form a number of commercial sources under the trade names such as CALGON Type "CPG", Type SGL, Type "CAL" and type "OL". Often such material is referred to as "activated" carbon or "activated" charcoal. Typically it is available in the form of an extremely fine, dusty particles having large surface areas (200 - several thousand m²/g.) It is to be understood that any of the "air purifying" or "activated" carbons of commerce can be used in the practice of this invention.

### Optional agents

The articles according to the present invention may further comprise other conventional agents like absorbent gelling materials.

### Absorbent gelling materials

As is well-known from recent commercial practice, absorbent gelling materials (sometimes referred to as "super-sorbers") are becoming broadly used in absorbent articles. AGM's are materials which have the twofold property of absorbing and retaining fluids and odours, and thus further contribute to the benefit of the present invention.

Such materials form hydrogels on contact with water (e.g., with urine, blood, and the like). One highly preferred type of hydrogel-forming, absorbent gelling material is based on polyacids, especially polyacrylic acid. Hydrogel- forming polymeric materials of this type are those which, upon contact with fluids (i.e., liquids) such as water or body fluids, imbibe such fluids and thereby form hydrogels. These preferred absorbent gelling materials will generally comprise substantially water-insoluble, slightly cross-linked, partially neutralized, hydrogel-forming polymer materials prepared from polymerizable, unsaturated, acid-containing monomers. In such materials, the polymeric component formed from unsaturated, acid-containing monomers may comprise the entire gelling agent or may be grafted onto other types of polymer moieties such as starch or cellulose. Acrylic acid grafted starch materials are of this latter type. Thus, the preferred absorbent gelling materials include hydrolyzed acrylonitrile grafted starch, acrylic acid grafted starch, polyacrylates, maleic anhydride-based copolymers and combinations thereof. Especially preferred absorbent gelling materials are the polyacrylates and acrylic acid grafted starch.

Whatever the nature of the polymer components of the preferred absorbent gelling materials, such materials will in general be slightly cross-linked. Crosslinking serves to render these preferred hydrogel-forming absorbent materials substantially water-insoluble, and cross-linking also in part determines the gel volume and extractable polymer characteristics of the hydrogels formed therefrom. Suitable cross-linking agents are well known in the art and include, for example, (1) compounds having at least two polymerizable double bonds; (2) compounds having at least one polymerizable double bond and at least one functional group reactive with the acid-containing monomer material; (3) compounds having at least two functional groups reactive with the acid-containing monomer materials; and (4) polyvalent metal compounds which can from ionic cross-linkages. Cross-linking agents of the foregoing types are described in greater detail in Masuda et al; U.S. Patent 4,076,663; Issued February 28, 1978. Preferred cross-linking agents are the di- or polyesters of unsaturated mono-or polycarboxylic acids with polyols, the bisacrylamides and the di-or triallyl amines. Especially preferred cross-linking agents are N,N'-methylenebisacrylamide, trimethylol propane triacrylate and triallyl amine. The cross-linking agent will generally comprise from about 0.001 mole percent to 5 mole percent of the preferred materials. More preferably, the cross-linking agent will comprise from about 0.01 mole percent to 3 mole percent of the gelling materials used herein.

The preferred, slightly cross-linked, hydrogel-forming absorbent gelling materials will generally be employed in their partially neutralized form. For purposes described herein, such materials are considered partially neutralized when at least 25 mole percent, and preferably at least 50 mole percent of monomers used to form the polymer are acid group-containing monomers which have been neutralized with a salt-forming cation. Suitable salt-forming cations include alkali metal, ammonium, substituted ammonium and amines. This percentage of the total monomers utilized which are neutralized acid group-containing monomers is referred to as the "degree of neutralization". Typically, commercial absorbent gelling materials have a degree of neutralization somewhat less than 90%.

The preferred absorbent gelling materials used herein are those which have a relatively high capacity for imbibing fluids encountered in the absorbent articles; this capacity can be quantified by referencing the "gel volume" of said absorbent gelling materials. Gel volume can be defined in terms of the amount of synthetic urine absorbed by any given absorbent gelling agent buffer and is specified as grams of synthetic urine per gram of gelling agent.

Gel volume in synthetic urine (see Brandt, et al, below) can be determined by forming a suspension of about 0.1-0.2 parts of dried absorbent gelling material to be tested with about 20 parts of synthetic urine. This suspension is maintained at ambient temperature under gentle stirring for about 1 hour so that swelling equilibrium is attained. The gel volume (grams of synthetic urine per gram of absorbent gelling material) is then calculated from the weight fraction of the gelling agent in the suspension and the ratio of the liquid volume excluded from the formed hydrogel to the total volume of the suspension. The preferred absorbent gelling materials useful in this invention will have a gel volume of from about 20 to 70 grams, more preferably from about 30 to 60 grams, of synthetic urine per gram of absorbent gelling material.

Another feature of the most highly preferred absorbent gelling materials relates to the level of extractable polymer material present in said materials. Extractable polymer levels can be determined by contacting a sample of preferred absorbent gelling material with a synthetic urine solution for the substantial period of time (e.g., at least 16 hours) which is needed to reach extraction equilibrium, by then filtering the formed hydrogel from the supernatant liquid, and finally by then determining the polymer content of the filtrate. The particular procedure used to determine extractable polymer content of the preferred absorbent gelling agent buffers herein is set forth in Brandt, Goldman and Inglin; U.S. Patent 4,654,039; Issues March 31,1987, Reissue 32,649, The absorbent gelling materials which are especially useful in the absorbent articles herein are those which have an equilibrium extractables content in synthetic urine of no more than about 17%, preferably no more than about 10% by weight of the absorbent gelling material.

The absorbent gelling materials herein before described are typically used in the form of discrete particles. Such absorbent gelling materials can be of any desired shape, e.g., spherical or semi-spherical, cubic, rod-like polyhedral, etc. Shapes having a large greatest dimension/smallest dimension ratio, like needles and flakes, are also contemplated for use herein. Agglomerates of absorbent gelling material particles may also be used.

The size of the absorbent gelling material particles may vary over a wide range. For reason of industrial hygiene, average particle sizes smaller than about 30 microns are less desirable. Particles having a smallest dimension larger than about 2mm may also cause a feeling of grittyness in the absorbent article, which is undesirable from a consumer aesthetics standpoint. Furthermore, rate of fluid absorption can be affected by particle size. Larger particles have very much reduced rates of absorption. Preferred for use herein are absorbent gelling material s particles substantially all of which have a particle size of from about 30 microns to about 2mm. "Particle Size" as used herein means the weighted average of the smallest dimension of the individual particles.

Typically, the articles like disposable absorbent articles herein may comprise absorbent gelling material particles at levels ranging from 10gm⁻² to 300gm⁻², preferably from 30gm⁻² to 150gm⁻², more preferably from 55gm⁻² to 85gm⁻².

### The disposable absorbent article

The odor control system (i.e., microorganisms as described herein and odour absorbing agent) may be incorporated into the absorbent article by any of the methods disclosed in the art, for example layered on the core of the absorbent article or mixed within the fibres of the absorbent core.

The microorganisms as described herein and the odour absorbing agent are preferably incorporated between two layers of cellulose tissue. Optionally the system may be bonded between two cellulose tissue layers with, for example, a hot melt adhesive or any suitable bonding system, as described in WO 94/01069.

In one embodiment of the present invention the microorganisms as described herein and the odour absorbing agent are incorporated in a layered structure in accordance with the disclosure of WO 94/01069 or Italian patent application number TO 93A 001028. TO 93A 001028 describes a layered structure substantially as described in WO 94/01069 with the exception that TO 93A 001028 comprises a much higher quantity of absorbent gelling material in the intermediate layer which is between the fibrous layers (120gm⁻²) that would be incorporated as an optional component in the present invention. The intermediate layer comprises in particular a polyethylene powder as thermoplastic material which is mixed with the odour absorbing agent and microorganisms. The mixture is then heated such that the polyethylene melts and glues the laminate layers together. Adhesive lines are preferably also placed on the edges of the laminate to ensure that the edges of the laminate stick and any loose microorganisms powder and odour absorbing agent present do not fall out of the laminate.

Alternatively, the polyethylene powder may be replaced by a conventional glue for instance those commercially available from ATO Findley under the name H20-31® to glue the laminate layers and/or components together. Advantageously this method step allows to avoid the heating step necessary when using polyethylene powder.

The microorganisms and the odour absorbing agent may be distributed homogeneously or non homogeneously over the entire absorbent article or in at least one layer of the topsheet or in at least one layer of the core or any mixture thereof. The microorganisms and the odour absorbing agent may be distributed homogeneously or non homogeneously on the whole surface of the desired layer or layers, or on one or several area of the surface layer/layers to which it is positioned (e.g. central area and/or surrounding area like the edges of a layer of the absorbent article) or mixtures thereof. Preferably the microorganisms herein are located in the core (also called intermediate layer which is positioned between the topsheet and the backsheet). The presence of the microorganisms in the core is preferred because the core collects and absorbs bodily fluids. Thus the close proximity to the substrate contributes to improved antagonistic activity of the microorganisms. Indeed optimum inhibition of the degradation activity by putrefactive bacteria and proliferation of pathogens is obtained.

The microorganisms and the odour absorbing agent may be positioned in different layers. For example in one embodiment herein the microorganisms are positioned such that at least a portion of the fluid discharge comes into contact with them before the odour absorbing agent (e.g., silica/zeolite). Preferably the microorganisms are located towards the topsheet or located in the topsheet itself (preferably the secondary topsheet) and the odour absorbing agent is located further away from the topsheet than the microorganisms. In one embodiment of the present invention, the microorganisms are positioned in at least one of the topsheet layers and the odour absorbing agent is positioned in the core.

In another embodiment the microorganisms and the odour absorbing agent may be located randomly together in various layers, i.e., that the total amount of microorganisms and odour absorbing agent is distributed in the topsheet layer and the core. The benefits of this execution are related with the combined action of microorganisms which inhibit the growth of degradative/pathogen bacteria in the core and on the body surface in contact with the absorbent article and of the odour absorbing agent that control odour in the head space and through the absorbent article.

The microorganisms as described herein and odour absorbing agent may be independently incorporated as a powder, typically when the spore form is used like lactospore® and when the living form is used in lyophilized form or freeze-dried. When used in a granulate or particulate form the microorganisms powder as described herein, the odour absorbing agents and optionally the absorbing gelling material may be granulated separately and then mixed together or granulated together.

Typical disposable absorbent articles according to the preferred embodiments of the present invention are those as described herein after:

### Absorbent core

According to the present invention, the absorbent can include the following components: (a) an optional primary fluid distribution layer preferably together with a secondary optional fluid distribution layer; (b) a fluid storage layer; (c) an optional fibrous ("dusting") layer underlying the storage layer; and (d) other optional components. According to the present invention the absorbent may have any thickness depending on the end use envisioned.

### a Primary/Secondary Fluid Distribution Layer

One optional component of the absorbent 'according to the present invention is a primary fluid distribution layer and a secondary fluid distribution layer. The primary distribution layer typically underlies the topsheet and is in fluid communication therewith. The topsheet transfers the acquired fluid to this primary distribution layer for ultimate distribution to the storage layer. This transfer of fluid through the primary distribution layer occurs not only in the thickness, but also along the length and width directions of the absorbent product. The also optional but preferred secondary distribution layer typically underlies the primary distribution layer and is in fluid communication therewith. The purpose of this secondary distribution layer is to readily acquire fluid from the primary distribution layer and transfer it rapidly to the underlying storage layer. This helps the fluid capacity of the underlying storage layer to be fully utilized. The fluid distribution layers can be comprised of any material typical for such distribution layers. In particular fibrous layers maintain the capillaries between fibers even when wet are useful as distribution layers.

### b Fluid Storage Layer

Positioned in fluid communication with, and typically underlying the primary or secondary distribution layers, is a fluid storage layer. The fluid storage layer can comprise any usual absorbent material or combinations thereof. It preferably comprises absorbent gelling materials in combination with suitable carriers.

Suitable carriers include materials which are conventionally utilized in absorbent structures such as natural, modified or synthetic fibers, particularly modified or non-modified cellulose fibers, in the form of fluff and/or tissues. Suitable carriers can be used together with the absorbent gelling material, however, they can also be used alone or in combinations. Most preferred are tissue or tissue laminates in the context of sanitary napkins and panty liners.

An embodiment of the absorbent structure made according to the present invention may comprise multiple layers comprises a double layer tissue laminate formed by folding the tissue onto itself. These layers can be joined to each other for example by adhesive or by mechanical interlocking or by hydrogen bridge bands. Absorbent gelling material or other optional material can be comprised between the layers.

Modified cellulose fibers such as the stiffened cellulose fibers can also be used. Synthetic fibers can also be used and include those made of cellulose acetate, polyvinyl fluoride, polyvinylidene chloride, acrylics (such as Orlon), polyvinyl acetate, non-soluble polyvinyl alcohol, polyethylene, polypropylene, polyamides (such as nylon), polyesters, bicomponent fibers, tricomponent fibers, mixtures thereof and the like. Preferably, the fiber surfaces are hydrophilic or are treated to be hydrophilic. The storage layer can also include filler materials, such as Perlite, diatomaceous earth, Vermiculite, etc., to improve liquid retention.

If the absorbent gelling material is dispersed non-homogeneously in a carrier, the storage layer can nevertheless be locally homogenous, i.e. have a distribution gradient in one or several directions within the dimensions of the storage layer. Non-homogeneous distribution can also refer to laminates of carriers enclosing absorbent gelling materials partially or fully.

### c Optional Fibrous ("Dusting") Layer

An optional component for inclusion in the absorbent core according to the present invention is a fibrous layer adjacent to, and typically underlying the storage layer. This underlying fibrous layer is typically referred to as a "dusting" layer since it provides a substrate on which to deposit absorbent gelling material in the storage layer during manufacture of the absorbent core. Indeed, in those instances where the absorbent gelling material is in the form of macro structures such as fibers, sheets or strips, this fibrous "dusting" layer need not be included. However, this "dusting" layer provides some additional fluid-handling capabilities such as rapid wicking of fluid along the length of the pad.

### d Other Optional Components of the absorbent structure

The absorbent core according to the present invention can include other optional components normally present in absorbent webs. For example, a reinforcing scrim can be positioned within the respective layers, or between the respective layers, of the absorbent core. Such reinforcing scrims should be of such configuration as to not form interfacial barriers to fluid transfer. Given the structural integrity that usually occurs as a result of thermal bonding, reinforcing scrims are usually not required for thermally bonded absorbent structures.

### The topsheet

According to the present invention the absorbent article comprises as an essential component a topsheet. The topsheet may comprise a single layer or a multiplicity of layers. In a preferred embodiment the topsheet comprises a first layer which provides the user facing surface of the topsheet and a second layer between the first layer and the absorbent structure/core.

The topsheet as a whole and hence each layer individually needs to be compliant, soft feeling, and non-irritating to the wearer's skin. It also can have elastic characteristics allowing it to be stretched in one or two directions. According to the present invention the topsheet may be formed from any of the materials available for this purpose and known in the art, such as woven and non woven fabrics and films.

In a preferred embodiment of the present invention at least one of the layers, preferably the upper layer, of the topsheet comprises a hydrophobic, liquid permeable apertured polymeric film. Preferably, the upper layer is provided by a film material having apertures which are provided to facilitate liquid transport from the wearer facing surface towards the absorbent structure. If present the lower layer preferably comprises a non woven layer, an apertured formed film or an airlaid tissue.

The term apertured polymeric topsheet as used herein refers to topsheets comprising at least one layer or a multiplicity of layers wherein at least one layer is formed from a continuous or uninterrupted film material wherein apertures are created. It has been surprisingly discovered that apertured polymeric film topsheets yield significantly better odor control than other types of topsheets such as for example thermal bonded nonwoven materials.

In general the apertured polymeric topsheet of the present invention is compliant, soft feeling, and non-irritating to the wearer's skin. Further, the topsheet is fluid pervious permitting fluids (e.g., menses and/or urine) to readily penetrate through its thickness. Suitable apertured polymeric film topsheets for use herein include polymeric apertured formed films, thermoplastic films, apertured plastic films, and hydroformed thermoplastic films; porous foams; net-like reticulated foams and thermoplastic films; and thermoplastic scrims.

Preferred topsheets for use in the present invention are selected from apertured formed film topsheets. Apertured formed films are especially preferred for the topsheet because they are pervious to body exudates and yet non-absorbent and have a reduced tendency to allow fluids to pass back through and rewet the wearer's skin. Thus, the surface of the formed film that is in contact with the body remains dry, thereby reducing body soiling and creating a more comfortable feel for the wearer. Suitable formed films are described in U.S. Patent 3,929,135 (Thompson), issued December 30, 1975; U.S. Patent 4,324,246 (Mullane, et al.), issued April 13, 1982; U.S. Patent 4,342,314 (Radel. et al.), issued August 3, 1982; U.S. Patent 4,463,045 (Ahr et al.), issued July 31, 1984; and U.S. 5,006,394 (Baird), issued April 9, 1991. Each of these patents are incorporated herein by reference. Particularly preferred microapertured formed film topsheets are disclosed in U.S. patent 4,609,518 (Curro et al), issue September 2, 1986 and U.S. patent 4,629,643 (Curro et al), issued December 16, 1986, which are incorporated by reference. The preferred topsheet for the present invention is the formed film described in one or more of the above patents and marketed on sanitary napkins by The Procter & Gamble Company of Cincinnati, Ohio as "DRI-WEAVE."

Suitable topsheets in the field of three dimensional formed film are described in EP 0 018 020 and EP 0 059 506. Especially preferred in a three dimensional formed polymeric topsheet having openings in the shape of regular pentagons which are regularly spaced and have an opening of 0.41 square millimeter. The openings are spaced 0.37 square millimeters apart transversely and 0.25 millimeters longitudinally. This topsheet has an initial opening (preforming ) thickness of about 25m a final (post forming) thickness of about 0.53mm and an open area of from 25% to about 40%.

Another formed film topsheet which is especially preferred is one having openings of two shapes; regular pentagons having an area of about 0.21 square millimeters and an irregular hexagon having an area of 1.78 square millimeters. The openings are distributed so that the distance between the sides of the figures is about 0.37 mm to about 0.42mm. The preforming and post forming film thickness are respectively 0.25 and 0.43 mm. This film has an open area of about 33.7%. Both films are made according to the teachings of the above mentioned patents.

A third form suitable topsheet comprises two separate perforated polymeric films superimposed one on the other.

The body surface of the formed film topsheet of the present invention may also be hydrophilic so as to help liquid to transfer through the topsheet faster than if the body surface was not hydrophilic. In this manner the likelihood that menstrual fluid will flow off the topsheet rather than flowing into and being absorbed by the absorbent structure is diminished. In a preferred embodiment, surfactant is incorporated into the polymeric materials of the formed film topsheet such as is described in U.S. Patent Application Serial No. 07/794,745, "Absorbent Article Having A Nonwoven and Apertured Film Coversheet" filed on November 19, 1991 by Aziz, et al., which is incorporated by reference. Alternatively, the body surface of the topsheet can be made hydrophilic by treating it with a surfactant such as is described in the above referenced U.S. 4,950,254, incorporated herein by reference.

### The backsheet

The backsheet primarily prevents the extrudes absorbed and contained in the absorbent structure from wetting articles that contact the absorbent product such as underpants, pants, pyjamas and undergarments. The backsheet is preferably impervious to liquids (e.g. menses and/or urine) and is preferably manufactured from a thin plastic film, although other flexible liquid impervious materials can also be used. As used herein, the term "flexible" refers to materials that are compliant and will readily conform to the general shape and contours of the human body. The backsheet also can have elastic characteristics allowing it to stretch in one or two directions.

The backsheet typically extends across the whole of the absorbent structure and can extend into and form part of or all of the preferred sideflaps, side wrapping elements or wings.

The backsheet can comprise a woven or nonwoven material, polymeric films such as thermoplastic films of polyethylene or polypropylene, or composite materials such as a film-coated nonwoven material. Preferably, the backsheet is a polyethylene film typically having a thickness of from about 0.012 mm (0.5 mil) to about 0.051 mm (2.0 mil).

Exemplary polyethylene films are manufactured by Clopay Corporation of Cincinnati, Ohio, under the designation P18-0401 and by Ethyl Corporation, Visqueen Division, of Terre Haute, Indiana, under the designation XP-39385. The backsheet is preferably embossed and/or matt finished to provide a more clothlike appearance. Further, the backsheet can permit vapors to escape from the absorbent structure, i.e. be breathable, while still preventing extrudates from passing through the backsheet. Also breathable backsheets comprising several layers, e.g. film plus non-woven structures, can be used.

Suitable breathable backsheets for use herein include all breathable backsheets known in the art. In principle there are two types of breathable backsheets, single layer breathable backsheets which are breathable and impervious to liquids and backsheets having at least two layers, which in combination provide both breathability and liquid imperviousness.

Suitable single layer breathable backsheets for use herein include those described for example in GB A 2184 389, GB A 2184 390, GB A 2184 391, US 4 591 523, US 3 989 867 US 3 156 242 and European Patent Application number 95120653.1.

Suitable dual or multi layer breathable backsheets for use herein include those exemplified in US 3 881 489, US 4 341 216, US 4 713 068, US 4 818 600, EPO 203 821, EPO 710 471, EPO 710 472, European Patent Application numbers 95120647.3, 95120652.3, 95120653.1 and 96830097.0.

Particularly preferred are backsheets meeting the requirements as defined in European Patent Application number 96830343.8 and more preferably wherein the absorbent article also meets the requirements as described therein.

According to the present invention the breathable backsheet comprises at least one, preferably at least two water vapor permeable layers. Suitable water vapor permeable layers include 2 dimensional, planar micro and macro-porous films, monolithic films, macroscopically expanded films and formed apertured films. According to the present invention the apertures in said layer may be of any configuration, but are preferably spherical or oblong. The apertures may also be of varying dimensions. In a preferred embodiment the apertures are preferably evenly distributed across the entire surface of the layer, however layers having only certain regions of the surface having apertures is also envisioned.

2 dimensional planar films as used herein have apertures having an average diameter of from 5 micrometers to 200 micrometers. Typically, 2-dimensional planar micro porous films suitable for use herein have apertures having average diameters of from 150 micrometers to 5 micrometers, preferably from 120 micrometers to 10 micrometers, most preferably from 90 micrometers to 15 micrometers. Typical 2 dimensional planar macroporous films have apertures having average diameters of from 200 micrometers to 90 micrometers. Macroscopically expanded films and formed apertured films suitable for use herein typically have apertures having diameters from 100 micrometers to 500 micrometers. Embodiments according to the present invention wherein the backsheet comprises a macroscopically expanded film or an apertured formed film, the backsheet will typically have an open area of more than 5%, preferably from 10% to 35% of the total backsheet surface area.

Suitable 2 dimensional planar layers of the backsheet may be made of any material known in the art, but are preferably manufactured from commonly available polymeric materials. Suitable materials are for example GORE-TEX (TM) or Sympatex (TM) type materials well known in the art for their application in so-called breathable clothing. Other suitable materials include XMP-1001 of Minnesota Mining and Manufacturing Company, St. Paul, Minnesota, USA. As used herein the term 2 dimensional planar layer refers to layers having a depth of less than 1mm, preferably less than 0.5mm, wherein the apertures have an average uniform diameter along their length and which do not protrude out of the plane of the layer. The apertured materials for use as a backsheet in the present invention may be produced using any of the methods known in the art such as described in EPO 293 482 and the references therein. In addition, the dimensions of the apertures produced by this method may be increased by applying a force across the plane of the backsheet layer (i.e. stretching the layer).

Suitable apertured formed films include films which have discrete apertures which extend beyond the horizontal plane of the garment facing surface of the layer towards the core thereby forming protuberances. The protuberances have an orifice located at their terminating ends. Preferably said protuberances are of a funnel shape, similar to those described in US 3, 929,135. The apertures located within the plane and the orifices located at the terminating end of protuberance themselves maybe circular or non circular, provided the cross sectional dimension or area of the orifice at the termination of the protuberance is smaller than the cross sectional dimension or area of the aperture located within the garment facing surface of the layer. Preferably said apertured preformed films are uni directional such that they have at least substantially, if not complete one directional fluid transport towards the core. Suitable macroscopically expanded films for use herein include films as described in for example in US 637 819 and US 4 591 523.

Suitable macroscopically expanded films for use herein include films as described in for example US 4 637 819 and US 4 591 523.

Suitable monolithic films include Hytrel™, available from DuPont Corporation, USA, and other such materials as described in Index 93 Congress, Session 7A "Adding value to Nonwovens", J-C. Cardinal and Y. Trouilhet, DuPont de Nemours International S.A., Switzerland.

According to the present invention the backsheet may comprise in addition to said water vapor permeable layer additional backsheet layers. Said additional layers may be located on either side of said water vapor permeable layer of the backsheet. The additional layers may be of any material, such as fibrous layers or additional water vapor permeable layers as described herein above.

### Odor control test

The odor reduction is measured by for example an in vitro sniff test. This test consists in analyzing the articles (including references) by expert graders that express their judgment about (un)pleasantness of the odor of the article (e.g.,pad).

The present invention is further illustrated by the following examples.

### Examples:

### Example A

The feminine pads used in the following examples were Always (Always is a registered Trade Mark) as sold by the Procter & Gamble Company.

Each feminine pad was opened by cutting the wrap around the perforated coverstock at its bottom face approximately along a longitudinal edge of the release paper which covers the external adhesive layer. The side of the absorbent fibrous core was then exposed by slightly shifting the water impermeable plastic bottom layer and subsequently, the fibrous core was split into two halves, each having approximately the same thickness, along a plane which is parallel to the plane of the napkin itself. The odor adsorbing agents (Silica or Zeolite or both) were homogeneously mixed together with lactic aid producing microorganisms and homogeneously distributed on this layer. Then all the layers were joined together to reconstitute the absorbent core.

The water impermeable inner backsheet was then put back into its original position and the wrap around perforated coverstock was sealed along the cut by means of e.g. a double sided adhesive tape.

Samples were produced using the method above, containing the odor control systems as described hereinbelow.

The lactic acid producing microorganisms used were *L. sporogenes* spores powder commercially available as Lactospore® (0.7g) from Sabinsa Corporation (Sochim International S.P.A. Milano). Thus each pad comprised about 10^{*}10⁹cfu of *L.sporogenes*. The silica (1g) used was Syloblanc 82 available from Grace GmbH. The zeolite (0.8g) used was Zeolite A, Wessalith CS, available from Degussa AG.

Alternatively other samples were prepared with *L. Sporogenes* (living form) commercially available under ATCC number 31284 in freeze-dried form instead of Lactospore®, the samples comprising about 10⁹cfu of such microorganisms.

### Example B

In Example B samples were produced using the same method as in Example A, except that AGM (0.8g) available from Dow Chemicals (XZ 9589001), was added to the lactic acid producing microorganisms and odor absorbing agents (silicate or zeolite or both).

### Example C

Other pads were prepared by following the method in example A except that after having split the fibrous core into two halves, the lactic acid producing microorganisms powder was homogeneously distributed onto the upper halve fibrous layer (i.e. the fibrous layer halve intended to be closer to the topsheet) and the odour absorbing agent (Silicate and/or Zeolite) was homogeneously distributed onto the lower halve fibrous layer (i.e., the one intended to be closer to the backsheet of the pad once reconstituted). Then a layer of airlaid tissue (19 mm^{*} 70 mm of low basis weight) (available from Fripa under the code/name NCB Tissue HWS) was positioned between the two halve fibrous layers which are then joined together to reconstitute the absorbent core. The presence of the airlaid tissue between the two fibrous layer avoids direct contact between the lactic acid producing microorganisms and the odor absorbing agents.

These samples were produced using as the lactic acid producing microorganisms powder, *L. sporogenes* spores powder commercially available as Lactospore® (0.7g) from Sabinsa Corporation (Sochim International S.P.A. Milano). Thus the pads comprised about 10 * 10⁹ cfu of *L.sporogenes*. The silica (0.8g) used was Syloblanc 82 available from Grace GmbH. The zeolite (0.8g) used was Zeolite A, Wessalith CS, available from Degussa AG.

Alternatively other samples were prepared with L. Sporogenes (living form) commercially available under ATCC number 31284 in freeze-dried form instead of Lactospore®, the samples comprising about 10⁹cfu of such microorganisms.

### Example D

In Example D samples were produced using the same method as in Example C, except that AGM (0.8g) available from Dow Chemicals (XZ 9589001), was added to the odor absorbing agents (silicate or zeolite or both) onto the lower halve fibrous layer.

All these pads were found to provide outstanding odour control benefits when in contact with bodily fluids. Indeed a synergistic odour reduction was observed when comparing these samples to samples containing only odour absorbing agents (zeolite and/or silicate) or only the microorganisms herein at same total level of total odour control materials.

## Claims

1. An article comprising as an odour control system for controlling odours, preferably odours associated with bodily fluids, lactic acid producing microorganisms together with at least one odour absorbing agent.

2. An article according to claim 1, wherein said lactic acid producing microorganisms are selected from the genera Lactobacillus, Lactococcus, Pedioccocus, Leuconostoc, Sporolactobacillus or a mixture thereof and more preferably from the species *Lactobacillus sporogenes*, *Sporolactobacillus inulinus, Lactobacillus acidophilus, Lactobacillus curvatus*, *Lactobacillus plantarum*, *Lactobacillus jenseni*, *Lactobacillus casei*, *Lactobacillus fermentum*, *Lactococcus lactis, Pediccocus acidilacti*, *Pediccocus pentosaceus*, *Pediccocus urinae* or *Leuconostoc mesenteroides*.

3. An article according to any of the preceding claims wherein the lactic acid producing microorganisms are spore forming lactic acid producing microorganisms and preferably the species *Lactobacillus sporogenes* in their living form or dormant form (spore).

4. An article according to any of the preceding claims wherein the article comprises more than 10² cfu, preferably more than 10⁴ cfu and more preferably from 10⁷ to 10¹⁰ cfu of said lactic acid producing microorganisms.

5. An article according to any of the preceding claims, wherein the odour absorbing agent is typically selected from the group consisting of silicas, zeolites, carbons, starches, cyclodextrine, kieselguhr, clays, ion exchange resins and combination thereof and preferably is a silicate, a zeolite or a combination thereof.

6. An article according to any of the preceding claims which comprises from 20 to 600 gm⁻², more preferably from 40 to 500 gm⁻², most preferably from 100 to 400 gm⁻², of the odour absorbing agent or a mixture thereof.

7. An article according to any of the preceding claims which comprises spores of lactic acid producing microorganisms, preferably of the species *Lactobacillus sporogenes*, together with both silica and zeolite as the odor absorbing agent.

8. An article according to any of the preceding claims which further comprises an absorbent gelling material.

9. An article according to claim 8, wherein the absorbent gelling material or a mixture thereof is present at a level from 10gm⁻² to 300gm⁻², preferably from 30gm⁻² to 150gm⁻², more preferably from 55gm⁻² to 85gm⁻².

10. An article according to any of the preceding claims wherein said article is a disposable absorbent article, preferably a sanitary napkin, pantiliner, tampon, diaper, incontinent pad, breast pad, perspiration pad, human or animal waste management device or interlabial pad.

11. An article according to any of the preceding claims wherein said article is an absorbent disposable article comprising a liquid pervious topsheet, a backsheet and an absorbent core intermediate said backsheet and said topsheet.

12. The use, as an odor control system, of lactic acid producing microorganisms, preferably spore-forming lactic acid-producing microorganisms and more preferably the species *Lactobacillus sporogenes*, together with an odour absorbing agent, preferably silicate and/or zeolite.
